# EUROPEAN PATENT APPLICATION

(11) **EP 4 495 231 A1**
(43) Date of publication of application: **22.01.2025**
(21) Application number: 23890266.2
(22) Date of filing: 26.07.2023
(51) Int. Cl.: C12N 9/10, C12P 19/26

(54) **MATURE POLYPEPTIDE SEQUENCE FOR SYNTHESIZING OLIGOSACCHARIDE AND USE**

(30) Priority: 19.11.2022 CN 202211449962
(71) Applicant: Shandong Henglu Biotech. Co., Ltd., Jinan, Shandong 250004 (CN)
(72) Inventor: FANG, Xu, Jinan, Shandong 250004 (CN); NIU, Kangle, Jinan, Shandong 250004 (CN); TANG, Qi, Jinan, Shandong 250004 (CN)
(74) Representative: Frick, Robert
(86) International application number: PCT/CN2023/109242
(87) International publication number: WO 2024/103825

(57) **Abstract**

The present invention provides a mature polypeptide sequence the amino acid sequence of which has at least 60%, or at least 70%, or at least 75%, or at least 80%, or at least 85%, or at least 90%, or at least 95%, or at least 97%, or at least 98%, or at least 99%, or 100% sequence identity to at least one of the sequences selected from SEQ ID NO: 1, SEQ ID NO: 2, SEQ ID NO: 3, SEQ ID NO: 4, SEQ ID NO: 5, SEQ ID NO: 6, SEQ ID NO: 7, SEQ ID NO: 8, SEQ ID NO: 9, and SEQ ID NO: 10. The mature polypeptide sequence has a relatively high capability to catalyze synthesis of lactose-N-tetrasaccharide.

## Description

The present invention claims priority to a Chinese patent application submitted to the Patent Office of the People's Republic of China on November 19, 2022, with application number 202211449962.6, titled "Mature Polypeptide Sequences for Synthesizing Oligosaccharide and Use", the entirety of which is incorporated herein by reference.

### TECHNICAL FIELD

The present invention relates to mature polypeptide sequences for synthesizing oligosaccharides and their expression in microorganisms, and belongs to the technical field of enzyme engineering/genetic engineering.

### BACKGROUND

Breast milk plays a crucial role in the growth and development of infants. Compared to breastfed infants, bottle-fed infants have lower survival rates and higher infection rates, sparking scientific interest in the components of breast milk. Since the 21st century, researchers have discovered that the total concentration of human milk oligosaccharides (HMOs, including acidic and neutral oligosaccharides) in colostrum can reach up to 24 g/L. However, this concentration decreases as lactation progresses. It has also been found that HMOs are vital for infant growth and long-term health, which can maintain intestinal microecological balance, promote the proliferation of beneficial bacteria in the gut, inhibit the growth of harmful bacteria, defend against pathogenic infections, regulate the immune system, foster cognitive development in infants, and etc. Many of the beneficial effects of breast milk are attributed to HMOs.

With continuous advancements in analytical tools and techniques for structural characterization, scientists are dedicated to identifying the components of breast milk, particularly the diverse and abundant HMOs. To date, over 200 types of HMOs have been identified. Among these, 2'-fucosyllactose (2'-FL), lacto-N-triose II (LNTII), lacto-N-tetraose (LNT), and lacto-N-neotetraose (LNnT) have been found to be the main components of HMOs, acting as bifidus factors. Research has demonstrated that adding HMOs to infant formula based primarily on cow's milk or goat's milk and feeding it to infants can show similar nutritional and immunological benefits of breastfeeding. Therefore, the addition of HMOs with physiological functions to infant formula, certain artificial milks, dairy products, baby foods, functional health products, and food for special populations will significantly enhance the physical health and quality of life of the respective groups, providing considerable social and economic value.

HMOs-added infant formula and dietary supplements are already available in markets in countries and regions such as the United States, the European Union, Australia, and New Zealand.

Currently, both the European Food Safety Authority and the United States Food and Drug Administration have approved the marketing of LNT, which is produced via microbial fermentation. The chemical structure of LNT is as follows:

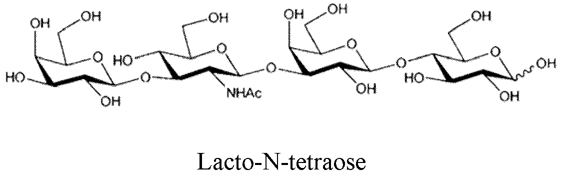

HMOs have been approved for use in various foods such as dairy products, baked goods, and beverages. The European Union has expanded the approved applications to include dietary supplements, formula foods for special medical purposes, and milk-based beverages or similar products intended solely for toddlers.

Given the limited supply of HMOs that meet the required standards, there is an urgent need for effective industrial-scale production. As extracting HMOs directly from natural breast milk is not an ideal approach, researchers have developed some chemical and enzymatic methods for producing HMOs. However, the production techniques disclosed in existing literature have drawbacks such as high production costs, low yields, and expensive prices. Chemical synthesis, although capable of producing structurally defined oligosaccharides through long multi-step protection-deprotection procedures, suffers from being labor-intensive, having low yields, and presenting food safety risks due to the use of heavy metals. Therefore, there is a pressing need for HMO synthesis technology that is stable, environmentally friendly, safe, cost-effective, highly efficient, and capable of large-scale industrial production to meet the public demand for HMOs.

The biosynthesis method has garnered widespread attention due to its advantages such as mild reaction conditions, short production cycle, high efficiency, environmental friendliness, and low cost. Patents such as CN201680052611.8, CN202180010201.8, and CN202180010202.2 have disclosed methods for synthesizing LNT using genetically modified *Escherichia coli* and other microbial host cells.

The biological method is favored for its mild reaction conditions, easy product separation, short production cycle, high efficiency, environmental friendliness, low cost and other advantages. Compared to the glycosylation reactions required in chemical synthesis, biosynthesis offers excellent stereoselectivity, and the product processing is relatively straightforward.

Enzymatic reactions are widely used for oligosaccharide synthesis due to their simplicity, specificity, and environmental friendliness. Japanese patent JP11253191A reports the synthesis of LNT using β-galactosidase with lactose-N-triose and galactose as substrates. Chinese patent CN201180071512.1 describes a method for producing LNnT and LNT from lactose-N-triose II using active cell extracts.

However, to achieve large-scale production of LNT via enzymatic synthesis and meet societal demand, the efficiency of enzymatic reactions needs to be further improved so that HMOs can be added to a wider range of food products, such as dairy products, baked goods, and beverages.

### SUMMARY

**Objectives:** In view of the shortcomings of existing technologies, the present invention provides a mature polypeptide sequence and its preparation method, as well as the use of these sequences for producing oligosaccharides specifically found in human breast milk. In particular, it focuses on the specific and efficient synthesis of lacto-N-tetraose (LNT) to improve existing techniques and enhance production efficiency.

Additionally, the present application provides nucleic acid products and enzyme products related to the polypeptide and their preparation methods and applications.

### Technical Solutions:

The present invention utilizes computer-aided design technology to screen for a mature polypeptide with the amino acid sequence as shown in SEQ ID NO:1 for the first time.

The mature polypeptide shown in SEQ ID NO:1 is a derivative of 1,3-beta-galactosyl-N-acetylhexosamine phosphorylase (also known as lacto-N-biose phosphorylase, LNBP).

The origins of the mature polypeptide with the amino acid sequence listed in SEQ ID NO:1 include *Aspergillus sp., Kluyveromyces* sp., *Bacillus sp.,* and *Bifidobacterium* sp., more preferably *Bifidobacterium Longum* or a microbe classified as *Bifidobacterium Longum,* which can be isolated from wild strains in nature, variant strains obtained through physical and chemical mutagenesis, or genetically modified engineered bacteria.

The present invention modifies at least one site in positions 236, 460, 572 and 608 of the mature polypeptide sequence shown in SEQ ID NO: 1 to obtain a mature polypeptide sequence with enhanced catalytic synthesis activity of LNT.

The modifications refer to any chemical alteration of the amino acid sequence constituting the polypeptide or its homologous sequences, as well as genetic manipulation of the DNA encoding the said polypeptide. The modifications can include the substitution, deletion, and/or insertion of one or more (several) amino acid(s), as well as the replacement of one or more (several) amino acid side chain(s).

Specifically, in a first aspect, the present invention relates to the said modifications selected from one or more combination(s) of the following (1)-(4) substitutions:
(1) Substituting cysteine at position 236 in the sequence shown in SEQ ID NO: 1 with tyrosine or valine;
(2) Substituting asparagine at position 460 in the sequence shown in SEQ ID NO:1 with isoleucine, valine, or methionine;
(3) Substituting aspartic acid at position 572 in the sequence shown in SEQ ID NO: 1 with valine or methionine;
(4) Substituting serine at position 608 in the sequence shown in SEQ ID NO:1 with valine or isoleucine.

The said more combinations involve two or more combinations.

The substitutions of one or more amino acid(s) are selected from the follows:
In the polypeptide sequence shown in SEQ ID NO:1, the cysteine at position 236 is substituted with tyrosine, resulting in the polypeptide with the amino acid sequence shown in SEQ ID NO:2.

In the polypeptide sequence shown in SEQ ID NO: 1, the asparagine at position 460 is substituted with isoleucine, valine, or methionine, resulting in the polypeptide with amino acid sequences shown in SEQ ID NO:3, SEQ ID NO:4, and SEQ ID NO:5, respectively.

In the polypeptide sequence shown in SEQ ID NO:1, the aspartic acid at position 572 is substituted with valine, resulting in the polypeptide with the amino acid sequence shown in SEQ ID NO:6.

In the polypeptide sequence shown in SEQ ID NO:1, the serine at position 608 is substituted with valine, resulting in the polypeptide with the amino acid sequence shown in SEQ ID NO:7.

In the polypeptide sequence shown in SEQ ID NO:1, the cysteine at position 236 is substituted with tyrosine, and simultaneously, the aspartic acid at position 572 is substituted with valine, resulting in the polypeptide with the amino acid sequence shown in SEQ ID NO:8.

In the polypeptide sequence shown in SEQ ID NO:1, the cysteine at position 236 is substituted with tyrosine, and simultaneously, the serine at position 608 is substituted with valine, resulting in the polypeptide with the amino acid sequence shown in SEQ ID NO:9.

In the polypeptide sequence shown in SEQ ID NO:1, the cysteine at position 236 is substituted with tyrosine, the asparagine at position 460 is substituted with methionine, and the serine at position 608 is substituted with valine, resulting in the polypeptide with the amino acid sequence shown in SEQ ID NO:10.

Preferably, the mature polypeptide sequences have amino acid sequences with at least 60%, or at least 70%, or at least 75%, or at least 80%, or at least 85%, or at least 90%, or at least 95%, or at least 97%, or at least 98%, or at least 99%, or 100% sequence identity to one or more of the sequence(s) shown in SEQ ID NO:2, SEQ ID NO:3, SEQ ID NO:4, SEQ ID NO:5, SEQ ID NO:6, SEQ ID NO:7, SEQ ID NO:8, SEQ ID NO:9, and SEQ ID NO:10.

In a second aspect, the present application provides a polynucleotide encoding the mature polypeptide sequences as described in the first aspect.

In a third aspect, the present application provides a nucleic acid construct comprising the polynucleotide described in the second aspect, and one or more regulatory sequence(s) that can be operatively linked to the polynucleotide. These regulatory sequence(s) can direct the production of the mature polypeptide sequence in an appropriate expression host.

In a fourth aspect, the present application provides an expression vector comprising the polynucleotide as described in the second aspect or the nucleic acid construct as described in the third aspect.

In a fifth aspect, the present application provides a transformed host cell, which has been transformed with the polynucleotide described in the second aspect, the nucleic acid construct described in the third aspect, or the expression vector described in the fourth aspect.

Preferably, the host cell is any one type of bacteria, molds, or yeasts;

The said recombinantly engineered host cells are selected from yeast cells, filamentous fungal cells, bacterial cells, etc.

Preferably, the bacterial cells include *Escherichia sp., Bacillus sp.,* etc.

The aforementioned *Escherichia sp.* is selected from *Escherichia coli, Escherichia hermannii,* and *Escherichia fergusonii.*

The aforementioned *Bacillus sp.* is selected from *Bacillus mycoides, Bacillus cereus, Bacillus polymyxa, Bacillus licheniformis, Bacillus brevis, Bacillus circulans,* and *Bacillus subtilis*; and more preferably, the aforementioned host cell is *Bacillus circulans* or *Bacillus subtilis.*

Preferably, the aforementioned filamentous fungal cells include *Aspergillus sp.* and *Trichoderma sp.*

The aforementioned *Aspergillus sp.* is selected from *Aspergillus oryzae, Aspergillus fumigatu, Aspergillus niger,* and *Aspergillus flavus*; and more preferably, the aforementioned host cell is *Aspergillus oryzae.*

The aforementioned *Trichoderma sp.* is selected from *Trichoderma reesei, Trichoderma viride,* and *Trichoderma koningii*; and more preferably, the aforementioned host cell is *Trichoderma reesei.*

Preferably, the aforementioned yeast cells include *Candida sp., Hansenula sp., Kluyveromyces sp., Pichia sp., Saccharomyces sp., Schizosaccharomyces sp.,* and *Yarrowia sp.;* more preferably, the host cells are selected from *Saccharomyces cerevisiae, Kluyveromyces lactis, Kluyveromyces marxianus, Yarrowia lipolytica, Rhodotorula graminis,Saccharomycespastorianus,* etc.; and far more preferably, the host cells are selected from *Saccharomyces cerevisiae, Kluyveromyces lactis, Kluyveromyces marxianus.*

In further preferred specific embodiments, the transformed host cells are genetically engineered bacteria involving the introduction of the polynucleotide described in the first aspect, or the nucleic acid construct described in the third aspect, or the expression vector described in the fourth aspect, which expresses the polypeptide as described in the first aspect. Preferably, the host cells include *Kluyveromyces lactis, Bacillus subtilis,* and *Aspergillus oryzae* containing the gene encoding the polypeptide as described in the first aspect.

Preferably, the aforementioned engineered bacteria include engineered strains of *Kluyveromyces lactis, Bacillus subtilis,* or *Aspergillus oryzae.*

In a sixth aspect, the present application provides an enzyme agent or composition containing the mature polypeptide sequence as described in the first aspect.

In a seventh aspect, the present application provides a method for producing the mature polypeptide sequence as described in the first aspect. The method includes:
(1) cultivating the transformed host cells under conditions suitable for expressing the mature polypeptide sequence; the transformed host cells are as described in the fifth aspect; and
(2) collecting the mature polypeptide.

In the specific embodiment, the aforementioned step (1) includes: firstly, introducing a nucleic acid construct or recombinant expression vector containing the gene encoding the mature polypeptide sequence as described in the first aspect into a host cell, constructing an engineered host cell that can express the polypeptide; and then, cultivating the engineered host cell and inducing the expression of the polypeptide.

In the specific embodiment, the step (2) includes isolating the polypeptide from the culture and purifying the isolated polypeptide.

Methods known in the art can be used to cultivate host cells in nutrient media suitable for producing the mature polypeptide sequence. For example, cells can be cultivated through shake flask cultures or in small-scale or large-scale fermentation (including continuous, batch, fed-batch, or solidstate fermentation) in laboratory or industrial fermenters using suitable media allowing the expression and/or the isolation of the mature polypeptide sequence. Using procedures known in the art, the cultivation occurs in appropriate nutrient media that contain sources of carbon and nitrogen, as well as inorganic salts. Suitable media can be purchased commercially or prepared according to disclosed compositions.

The polypeptide can be recovered from the culture using methods known in the art. For example, the polypeptide variant can be recovered from nutrient media through various conventional procedures, including but not limited to collection, centrifugation, filtration, extraction, spray drying, evaporation, or precipitation.

The mature polypeptide sequence in the present invention can be purified using methods known in the art to obtain a pure mature polypeptide. The said methods for isolation or purification include but are not limited to chromatography (e.g., ion exchange chromatography, affinity chromatography, hydrophobic interaction chromatography, and gel filtration chromatography), electrophoresis (e.g., preparative isoelectric focusing), differential solubility (e.g., ammonium sulfate precipitation), sodium dodecyl sulfate-polyacrylamide gel electrophoresis, salt fractionation, etc., or their combinations. More preferably, purification can be conducted using nickel (Ni) column affinity chromatography.

In a eighth aspect, the present application provides a method for preparing LNT, which includes: use the mature polypeptide as described in the first aspect, or the transformed host cells as described in the fifth aspect (particularly the transformed engineered bacteria), or the enzyme agent or composition as described in the sixth aspect as a catalyst, and lactose and its derivatives as substrates to produce LNT through catalytic reactions or fermentation reactions.

In the method above, preferably, the transformed host cells include *Kluyveromyces lactis, Bacillus subtilis,* or *Aspergillus oryzae* transformed with the polynucleotide as described in the second aspect, or the nucleic acid construct as described in the third aspect, or the expression vector as described in the fourth aspect.

Preferably, in the preparation method above, the reaction substrates are selected from galactose-1-phosphate and lacto-N-triose II (LNTII), or lactose, or galactose, or N-acetylglucosamine, or carbohydrates containing an N-acetylglucosamine group; more preferably, the reaction substrates are galactose-1-phosphate and LNTII.

Preferably, the reaction solvent is an aqueous solvent or a solution containing solids.

In specific embodiments, the purified form of the polypeptide as described in the first aspect, or the culture containing said polypeptide (i.e., crude enzyme solution) can be used; additionally, the polypeptide can be used in an immobilized form, or in the form of an enzyme agent mixed with other materials that can maintain or enhance the polypeptide's activity.

Furthermore, the process parameters such as reaction temperature, the amount of catalyst added, reaction time, and pH can be optimized or determined according to the methods disclosed in the embodiments of the present application.

In a ninth aspect, the present application provides the use of the polypeptide as described in the first aspect, the transformed host cells as described in the fifth aspect (particularly the transformed engineered bacteria), or the enzyme agent or composition as described in the sixth aspect as a catalyst in the production of lacto-N-tetraose (LNT).

Preferably, the said transformed host cells include *Kluyveromyces lactis, Bacillus subtilis,* or *Aspergillus oryzae* transformed with the polynucleotide as described in the second aspect, or the nucleic acid construct as described in the third aspect, or the expression vector as described in the fourth aspect.

Preferably, the method for producing LNT in the use above is the method as described in the eighth aspect.

### Term explanation:

The term "Sequence Identity": Correlation between two amino acid sequences, described by the parameter of "sequence identity".

The term "Expression" refers to any step involved in the production of the mature polypeptide, including but not limited to, transcription, post-transcriptional modification, translation, post-translational modifications, etc. Expression can be measured using techniques known in the art, such as measuring the concentration or activity of mRNA and/or the translated mature polypeptide.

The term "Host Cell" refers to any cell type that is susceptible to transformation, transfection, and transduction with an expression vector. The term "host cell" encompasses any progeny of the parent cell that is different from the parent cell due to substitution occurring during cell replication.

The term "Mature Polypeptide" refers to a polypeptide in its final form after translation and any post-translational modifications, such as N-terminal processing, C-terminal truncation, etc. In an aspect herein, the mature polypeptide includes a protein with the amino acid sequence(s) shown in SEQ ID NO:1-10.

### Benefits

The present invention provides a series of mature polypeptide sequences with high catalytic activity for synthesizing lacto-N-tetraose (LNT), which exhibit excellent thermal stability and have promising applications. Although existing technologies have explored the use of certain enzymes for the catalytic synthesis of HMOs, the low catalytic efficiency, difficulty in obtaining substrates, high prices of substrates, or poor reaction specificity leads to many problems such as complex downstream isolation and purification processes.

The present invention provides methods for expressing mature polypeptide sequences capable of producing LNT and methods for the catalytic synthesis of LNT using the said mature polypeptide sequences. In the present invention, the existing mature polypeptide sequences are optimized via genetic engineering technology, and the obtained mature polypeptide sequences have the enhanced activity for the catalytic synthesis of LNT.

The technical solutions of the present invention are of significant importance for the industrial production of HMOs as an environmentally friendly, efficient, and sustainable method for large-scale industrial applications.

### BRIEF DESCRIPTION OF THE DRAWINGS

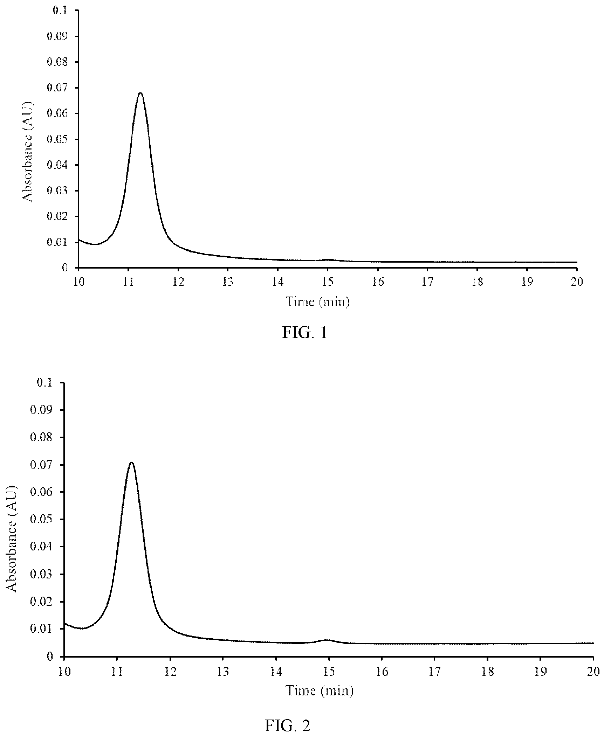

FIG. 1 depicts the HPLC chromatogram of a purified reaction mixture obtained from the catalytic synthesis of LNT by LNBP-M3 in Example 2.
FIG. 2 illustrates the HPLC chromatogram of a purified reaction mixture obtained from the catalytic synthesis of LNT by LNBP-M3-C236Y/D572V in Example 2.

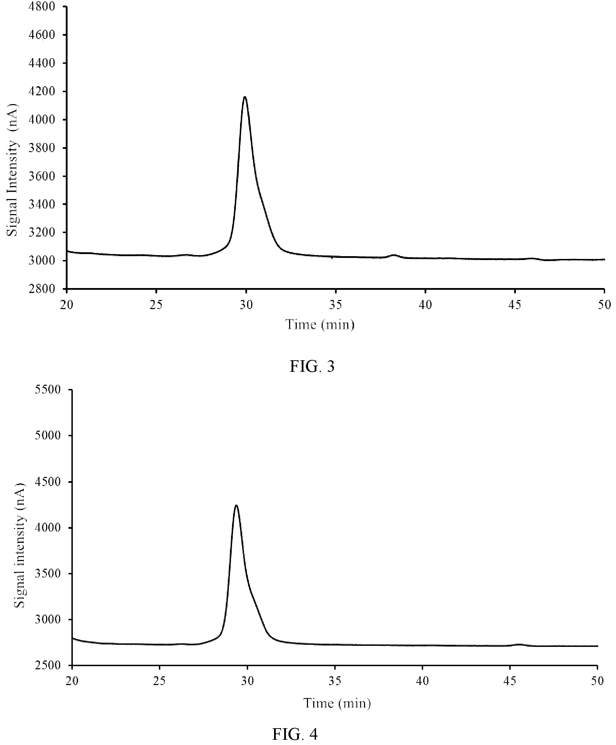

FIG. 3 shows the ion chromatogram of the substance near rt = 11.34 in the HPLC chromatogram as shown in FIG. 1.
FIG. 4 is the ion chromatogram of a reaction mixture of the substance at rt = 11.34 in the HPLC chromatogram as shown in FIG. 2.
FIG. 5 displays the liquid chromatography-mass spectrometry (LC-MS) image of the substance at rt = 11.34 in the HPLC chromatogram as shown in FIG. 1.

### DETAILED DESCRIPTION

The experimental methods used in the following examples are conventional ones unless otherwise specified. All materials and reagents are commercially available unless otherwise noted.

The following examples are provided to further illustrate the present invention in detail. It should be understood that the specific embodiments described herein are merely for explaining the present invention and are not intended to limit the scope of the present invention. Modifications or replacements of technical details and forms within the scope of structural concept and use of the present invention fall within the scope of protection.

Unless otherwise defined, all technical and scientific terms used herein have the same meaning as commonly understood by any person skilled in the art. Generally, the nomenclature used and the experimental methods described below are well-known and conventionally employed in the art.

In the following examples, the products obtained from the catalytic reaction by the mature polypeptide are analyzed or identified using HPLC, LC-MS, and ion chromatography.

The HPLC system parameters are as follows:
Chromatographic column model: Cosmosil Sugar-D amino column (nacalaitesque, INC.); Detector: UV detector (Chromaster, Hitachi); Detection wavelength: 210 nm; Injection volume: 10 µL; Flow rate: 1.0 mL/min; Column temperature: 30°C; Mobile phase: acetonitrile:water=70:30.

The LC-MS system parameters are as follows:
Chromatographic column model: Cosmosil Sugar-D amino column (nacalaitesque, INC.); Detector: UV detector (Chromaster, Hitachi); Detection wavelength: 210 nm; Injection volume: 10 µL; Flow rate: 1.0 mL/min; Column temperature: 30°C; Mobile phase: acetonitrile:water=70:30; H-EIS mode, molecular weight scan range: 400-900.

The IC system parameters are as follows: Chromatographic column model: MetroSep Carb 2 (4.0 mm × 250 mm); Eluent: 140 mM NaOH/20 mM NaAc, isocratic elution; Flow rate: 0.500 mL/min; Amperometric detector; Column temperature: 40°C; Injection volume: 20 µL; Run time: 50 min.

The standard preparations of LNTII, LNT, and LNnT are sourced from ELICITYL, France.

It should be noted that the terms used herein are intended to describe specific embodiments and are not meant to limit the exemplary embodiments of the present invention. Additionally, it should be understood that the terms "comprising" and/or "including" used in the patent specification indicate the presence of stated features, steps, operations, devices, components, and/or their combinations. It should be understood that the scope of protection of the present invention is not limited to the specific examples detailed below. Furthermore, it should also be understood that the terms used in the examples are meant to describe specific examples, not to limit the scope of the present invention. Unless specified otherwise, the experimental methods described in the following embodiments, where specific conditions are not noted, are generally conducted according to conventional molecular biology techniques and conditions known in the art. These techniques and conditions are fully explained in existing literature. Refer to, for example, Sambrook et al., Molecular Cloning: A Laboratory Manual*,* for detailed descriptions of techniques and conditions, or refer to the conditions recommended by the manufacturers.

### Example 1. Expression of Mature Polypeptide Sequences (SEQ ID NO: 1-10) in Escherichia coli

A gene synthesis company was commissioned to obtain genes corresponding to the amino acid sequences of SEQ ID NO: 1-10 through codon optimization and DNA synthesis. Specifically, based on the nucleotide sequence shown in SEQ ID NO:25, the genes encoding the polypeptides shown in SEQ ID NO: 1-10 were obtained through polymerase chain reaction (PCR) amplification using the primers listed in Table 1.

Following the methods described in *Molecular Cloning: A Laboratory Manual,* a series of pET32a-lnbpX recombinant plasmids (pET32a-lnbp1-10) were constructed, as detailed in Table 1. The recombinant plasmids above were then transformed into *Escherichia coli* BL21(DE3) according to the following steps: The prepared *Escherichia coli* BL21(DE3) competent cells were taken and placed on ice for 30 min to thaw. Then, 100 µL of the competent cells were taken and mixed with 10 µL of pET32a-lnbpX recombinant plasmids at a concentration of 50 ng/µL, and were heat shocked at 42°C for 45 s, followed by immediate cooling on ice for 2 min. Then, 1 mL of fresh lysogeny broth (LB) medium (containing 1.0% tryptone, 0.5% yeast extract, and 1.0% sodium chloride (NaCl), with 1.5% agar powder added to the plate) was added to incubate at 37°C and 100 rpm for 1 h until recovery. Subsequently, 100 µL of the cell suspension was plated on an LB plate containing ampicillin (100 µg/mL) and incubated at 37°C for 12 h. After that, single colonies were selected for colony PCR (see Table 1 for PCR primers) to identify positive transformants. Further, the plasmids from correctly transformed colonies were extracted and verified by double digestion and gene sequencing to confirm whether the pET32a-lnbpX recombinant plasmids were successfully introduced into *Escherichia coli.*

Next, the correctly transformants were inoculated into LB liquid medium and incubated at 37°C and 200 rpm in a shaker for 12 h to obtain the seed culture. The seed culture was then inoculated into fresh LB medium at 1% (v/v) and incubated at 37°C until OD₆₀₀ reached 0.8, was induced by isopropyl-β-D-thiogalactopyranoside (IPTG) at a final concentration of IPTG 0.1 mmol/L, and was incubated at 16°C and 200 rpm for 12 h. After inducing expression, the fermentation broth was centrifuged at 4°C and 5,000 rpm for 30 min to collect the cell pellet. The cell pellet was resuspended in 20 mM phosphate-buffered saline (pH 7.4), followed by ultrasonic disruption at a frequency of plus on 5s/ off 5s for 30 min. The disrupted cell suspension was then centrifuged at 13,000 ×g and 4°C for 30 min to remove cell debris, and the supernatant was collected.

The soluble mature polypeptides were purified using Ni column affinity chromatography as follows: Deionized water was added to the top of the Ni column to elute naturally. Then, the column was washed with five-column volumes of a binding buffer. The crude enzyme solution was filtered through a 0.45 µm filter and loaded onto the column at a flow rate of 1.5 mL/min to ensure adequate binding of the sample to the Ni column. After the sample has drained, the column was washed with five-column volumes of a washing buffer in a continuous gradient to remove contaminant proteins. Finally, the target protein was eluted with five-column volumes of an elution buffer, and the eluate was collected. The expression of the target protein was then analyzed using SDS-PAGE. After the genetically engineered bacteria were induced, the distinct specific expression band was observed, and the molecular weight of the band was basically consistent with the expected molecular weight of 103 kDa. The obtained proteins correspond to the mature polypeptide sequences shown in SEQ ID NO: 1-10, as detailed in Table 2.

**Table 1. Primers for recombinant plasmids**

| Recombinant plasmids | Degenerate primers and DNA sequences | Primer DNA sequence number |
|---|---|---|
| pET32a-lnbp 1 | ATG GGT GGA AAC AAG CAG ACG TAT TCG TAT TAC GGC ATT C | SEQ ID NO: 11 |
| | GAA CGC CAT CCA CGA ACG CAT CTT GCC | SEQ ID NO: 12 |
| pET32a-lnbp2 (C236Y) | F:TTCGGCTACGCCTGCACCGTGAGCCCGGCCGCG | SEQ ID NO: 13 |
| | R:GCAGGCGTAGCCGAACCAGTCCACGACCTTCTCGC GCTG | SEQ ID NO: 14 |
| pET32a-lnbp3 (N460I) | F:GGGTGGAATCAAGCAGACGTATTCGTATTACGGCAT | SEQ ID NO: 15 |
| | R:CTGCTTGATTCCACCCATGAACGCCATCCACG | SEQ ID NO:16 |
| pET32a-lnbp4 (N4604M) | F: GGGTGGAGTC AAGC AGACGTATTCGTATTACGGC AT | SEQ ID NO:17 |
| | R:CTGCTTGACTCCACCCATGAACGCCATCCACG | SEQ ID NO: 18 |
| pET32a-lnbp5 (N460V) | F: GGGTGGAATGAAGC AGACGTATTCGTATTACGGC AT | SEQ ID NO:19 |
| | R:CTGCTTCATTCCACCCATGAACGCCATCCACG | SEQ ID |
| | | NO:20 |
| pET32a-lnbp6 (D572V) | F:GTCGGTGGTCAAGTACTTCCCGGCCGTCACGCCG | SEQ ID NO:21 |
| | R:GTACTTGACCACCGACAGCGTCTGGTAGCGTTCCT CGTC | SEQ ID NO:22 |
| pET32a-lnbp7 (S608V) | F:CCGCTGGTGGGCTGCGGCGGTGGCCAGGGCATC | SEQ ID NO:23 |
| | R:GCAGCCCACCAGCGGGATGCGGTATCCGGCCTTCT CCCACG | SEQ ID NO:24 |
| pET32a-lnbp8 (C236Y/D572 V) | Same as pET32a-lnbp3 (C236Y), pET32a-lnbp7 (D572V) | N/A |
| pET32a-lnbp9 (C236Y/S608 V) | Same as pET32a-lnbp 3(C236Y), pET32a-lnbp8 (S608V) | N/A |
| pET32a-lnbp 10 (C236Y/N460 M /S608V) | Same as pET32a-lnbp3 (C236Y), pET32a-lnbp5 (N4604M), pET32a-lnbp8 (S608V) | N/A |

**Table 2. Mature polypeptides and their corresponding plasmids and amino acid sequence number**

| Plasmids | Mature polypeptides | Amino acid sequence number |
|---|---|---|
| pET32a-lnbp1 | LNBP-M3 | SEQ ID NO: 1 |
| pET32a-lnbp2 | LNBP-M3-C236Y | SEQ ID NO:2 |
| pET32a-lnbp3 | LNBP-M3-N460I | SEQ ID NO:3 |
| pET32a-lnbp4 | LNBP-M3-N460V | SEQ ID NO:4 |
| pET32a-lnbp5 | LNBP-M3-N460M | SEQ ID NO:5 |
| pET32a-lnbp6 | LNBP-M3-D572V | SEQ ID NO:6 |
| pET32a-lnbp7 | LNBP-M3-S608V | SEQ ID NO:7 |
| pET32a-lnbp8 | LNBP-M3 -C23 6Y/D5 72V | SEQ ID NO:8 |
| pET32a-lnbp9 | LNBP-M3-C236Y/S608V | SEQ ID NO:9 |
| pET32a-lnbp10 | LNBP-M3-C236Y/ N460M /S608V | SEQ ID NO:10 |

### Example 2. Catalytic Synthesis of LNT Using Mature Polypeptide Sequences (SEQ ID NO:1-10)

The specific operations were as follows: 20 mM Gal-1-P, 20 mM LNTII, 1 mmol/L MgCl₂, 2.2 mg/mL polypeptide (each corresponding to SEQ ID NO:1-10), and 100 mM tris(hydroxymethyl)aminomethane hydrochloride buffer (pH 7) were thoroughly mixed to react for 24 h at 37°C. Then the reaction was terminated, and the product was purified using gel column chromatography. The catalytic reaction products of the mature polypeptide sequences were analyzed and identified using HPLC, IC, and LC-MS.

The HPLC system parameters are as previously described. The results are presented below:
(1) The LNTII standard preparation showed a peak retention time (rt) of 8.79 min, and the LNT and LNnT standard preparations both showed a peak rt of 11.34 min
(2) The reaction mixtures of mature polypeptide sequences SEQ ID NO: 1-10 all exhibited strong absorption peaks around 11.34 min, consistent with the rt values of the LNT and LNnT standard preparations.

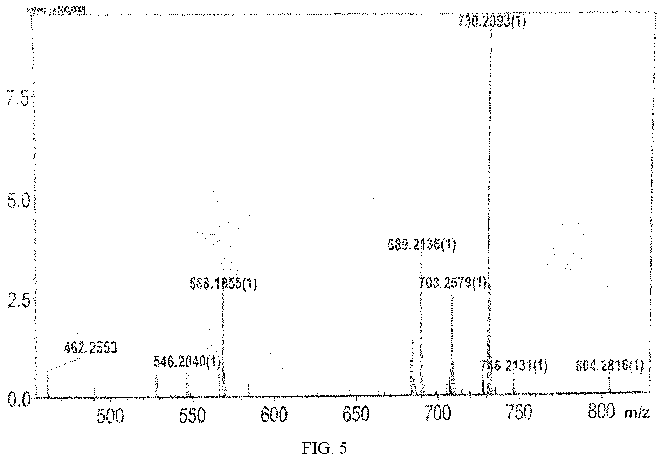

The LC-MS system parameters and results are as follows:
The LC-MS analysis was used to analyze the product at rt = 11.34 min from the HPLC chromatogram of the LNBP-M3 reaction mixture, shown in FIG. 5. The molecular weight for LNT+/H is 708.2579 and for LNT+/Na is 730.2393. The MS results matched the following: Compound CID: 440993; Chemical formula: C₂₆H₄₅NO₂₁; Extracted mass: 707.63, the molecular weights of the product are consistent with those of LNT and LNnT.

The IC system parameters are as previously described. The results are given below:
(1) The LNTII standard preparation showed a peak rt of 16.9 min; the LNnT standard preparation showed a peak rt of 25.7 min; and the LNT standard preparation showed a peak rt of 29.8 min.
(2) After the reaction solution of LNBP-M3 was purified, a strong absorption peak was observed near 29.8 min, consistent with the peak rt of the LNT standard preparation, indicating successful synthesis of LNT. No absorption peak was observed near 25.7 min, indicating that LNnT was not synthesized.

After the reaction mixtures of mature polypeptide sequences (SEQ ID NO:2-10) were purified separately, they were analyzed using the same process as LNBP-M3 (SEQ ID NO:1). The results demonstrated that, similar to the LNBP-M3 reaction mixture, the mature polypeptide sequences (SEQ ID NO:2-10) catalyzed the synthesis of LNT, but no LNnT was synthesized.

### Conclusion

The HPLC, IC, and LC-MS findings collectively show that the mature polypeptide sequences shown in SEQ ID NO:2-10 catalyze the synthesis of LNT from the substrates Gal-1-P and LNTII, but do not synthesize LNnT.

The measured LNT concentrations in the reaction mixtures are shown in Table 3.

**Table 3. The results of LNT synthesized by the catalysis of the mature polypeptide shown in SEQ ID NO: 1-10**

| Mature polypeptides | Concentration of LNT, mg/mL |
|---|---|
| LNBP-M3 | 6.79 |
| LNBP-M3-C236Y | 0.6 |
| LNBP-M3- N460I | 8.66 |
| LNBP-M3- N460V | 7.46 |
| LNBP-M3- N460M | 8.28 |
| LNBP-M3-D572V | 1.07 |
| LNBP-M3- S608V | 9.91 |
| LNBP-M3- C236Y/D572V | 8.53 |
| LNBP-M3- C236Y/S608V | 10.73 |
| LNBP-M3- C236Y/ N460M /S608V | 10.87 |

The data in Table 3 demonstrate that LNBP-M3-N4601, LNBP-M3-N460V, LNBP-M3-N460M, LNBP-M3-S608V, LNBP-M3-C236Y/D572V, LNBP-M3-C236Y/S608V, and LNBP-M3-C236Y/N460M/S608V show higher catalytic activity in the synthesis of LNT compared to the original LNBP-M3 protein.

### Example 3. Effect of Temperature on the Catalytic Activity of Mature Polypeptide Sequences (SEQ ID NO:1-10)

Using the catalytic synthesis method described in Example 2, the reaction system was placed in a water bath at temperatures of 10°C, 25°C, 37°C, 40°C, 45°C, 50°C, 60°C, 70°C, and 80°C for 24 h, respectively. The yield of LNT in the reaction mixture was measured using HPLC, and the results are recorded in Table 4.

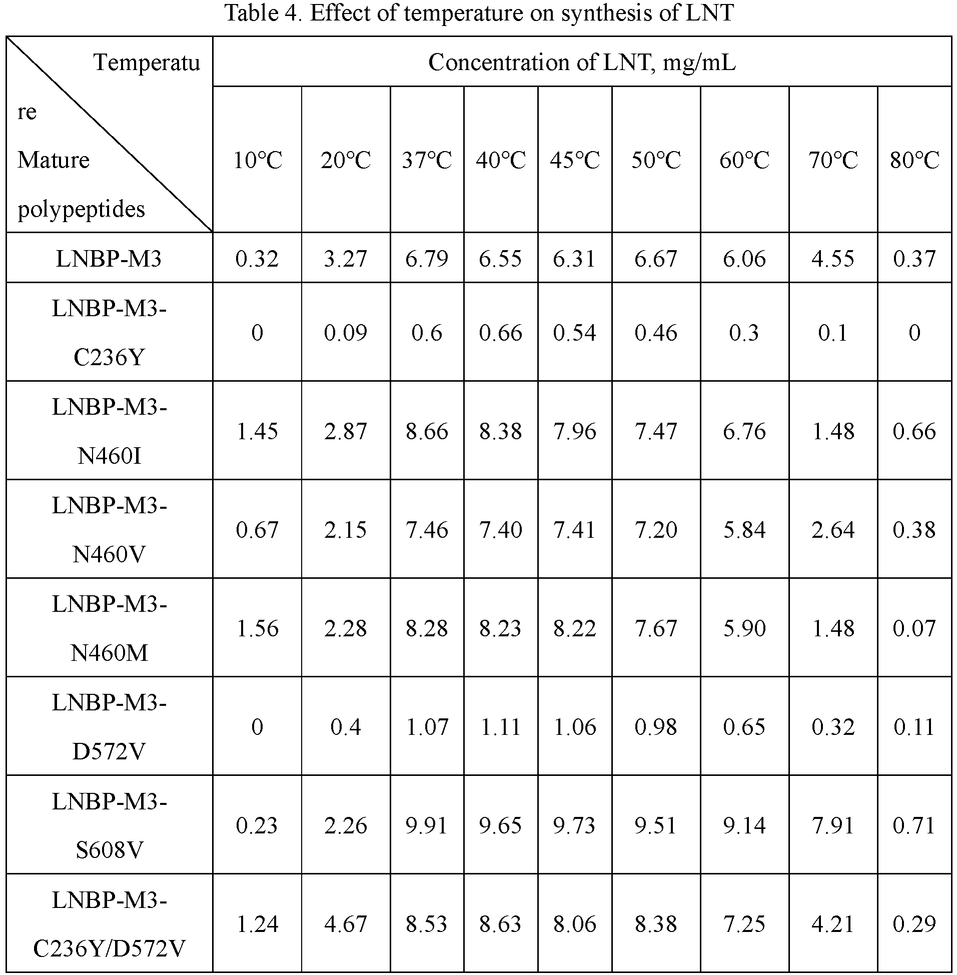

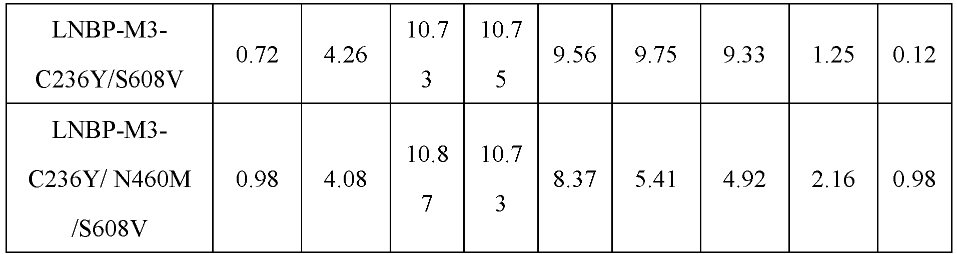

The data in Table 4 are interpreted as follows:
(1) In the temperature range of 10-60°C, the polypeptide sequences shown in SEQ ID NO: 1-10 all demonstrated catalytic activity in the synthesis of LNT. Among them, LNBP-M3-S608V, LNBP-M3-C236Y/D572V, and LNBP-M3-C236Y/S608V showed basically unchanged catalytic activities in synthesizing LNT in the temperature range of 37-60°C, indicating excellent thermal stability. This is significant for their broader application and commercialization.
(3) LNBP-M3-N4601, LNBP-M3-N460V, and LNBP-M3-N460M exhibited over 60% of their catalytic activities at 60°C (24 h) compared to their activities at 37°C (24 h), suggesting good heat resistance.

### Example 4. Expression of Mature Polypeptides SEQ ID NO:1 (LNBP-M3) and SEQ ID NO:8 (LNBP-M3-C236YID572V) in Kluyveromyces lactis (CICC1773)

The construction of recombinant plasmid pKLAC1-LNBP-M3 or pKLAC1-LNBP-M3-C236Y/D572V is described as follows:
Based on the amino acid sequences of mature polypeptides SEQ ID NO:1 and SEQ ID NO:8, codon optimization and DNA synthesis were performed to construct the expression plasmid KLAC1-LNBP-M3 or KLAC1-LNBP-M3-C236Y/D572V.

Subsequently, the expression plasmid KLAC1-LNBP-M3 or KLAC1-LNBP-M3-C236Y/D572V was then transformed into *Kluyveromyces lactis,* and the transformation method is as follows:
(1) Preparation of *Kluyveromyces lactis* competent cells: A small amount of the yeast strain stock was streaked on a solid agar plate and incubated upside down at 30°C for 2 days. A single yeast colony was selected and inoculated into 50 mL of liquid culture medium at 30°C and 220 rpm until the OD₆₀₀ reached 0.8-1.5. A cell pellet was collected and washed with 25 mL of sterile water, followed by centrifugation at 1,500 ×g for 10 min at room temperature, and the supernatant was discarded. The pellet was resuspended in 1 mL of 100 mM lithium chloride buffer and centrifuged at 12,000 rpm for 30s, and the supernatant was discarded. The pellet was resuspended again in 400 µL of 100 mM lithium chloride buffer to obtain competent yeast cells. Then, the yeast cells were aliquoted into 50 µL per tube for transformation.
(2) Transformation: The prepared competent yeast cells were centrifuged, and the residual lithium chloride solution was removed using Tips. For each transformation, the following substances were added in sequence: 50% PEG3350 (240 µL), 1 M LiCl (36 µL), 2 mg/mL single-stranded Salmon sperm DNA (25 µL), and 5-10 µg/50 µL H₂O plasmid DNA (50 µL) and vigorously mixed by vortex mixer until the yeast cells were evenly suspended. The mixture was incubated in a 30°C water bath for 30 min, and then heat shocked in a 42°C water bath for 20-25 min, followed by centrifugation at 8,000 rpm for 10 min, and the yeast cells were harvested. The yeast cells were then resuspended in 500 µL of YPD liquid medium and incubated on a shaker at 30°C for 1-4 h. Subsequently, 25-100 µL of the yeast culture was plated on G418-resistant selective media and incubated upside down at 30°C for 2-3 days until the appearance of the target transformants, namely the *Kluyveromyces lactis* recombinant strains.

The said plasmid DNA is either KLAC1-LNBP-M3 or KLAC1-LNBP-M3-C236Y/D572V expression plasmid. The process of fermentation in the shake flask is described as follows: 1) Preparation of primary seed culture: A single colony of the target *Kluyveromyces lactis* recombinant strain was inoculated into 10 mL of YGD liquid medium to incubate overnight at 30°C and 200 rpm to obtain the primary seed. 2) Preparation of secondary seed culture: The primary seed culture was inoculated into 1 L of YGD liquid medium to incubate at 30°C and 200 rpm until the OD600 reaches approximately 7. 3) The YGD liquid medium was centrifuged to obtain a crude enzyme solution containing the target protein. Then, the target protein was purified according to the method described in Example 1. The resulting protein is either KL-LNBP-M3 (SEQ ID NO:1) or KL-LNBP-M3-C236Y/D572V (SEQ ID NO:8).

KL-LNBP-M3 or KL-LNBP-M3-C236Y/D572V, obtained from this example, was used for the catalytic synthesis as described in Example 2. The reaction system was placed in a water bath at 37°C for 24 h. The yield of LNT in the reaction mixture was measured using HPLC, and the results are recorded in Table 5.

**Table 5. Catalytic synthesis of LNT by KL-LNBP-M3 and KL-LNBP-M3-C236Y/D572V expressed by kluyveromyces lactis**

| Recombinant protein | Concentration of LNT, mg/mL |
|---|---|
| KL-LNBP-M3 | 6.42 |
| KL-LNBP-M3- C236Y/D572V | 8.01 |

### Example 5. Expression of Mature Polypeptides SEQ ID NO:1 (LNBP-M3) and SEQ ID NO:8 (LNBP-M3-C236Y/D572V) in Bacillus subtilis (Bacillus subtilis 168)

Following the methods outlined in *Molecular Cloning: A Laboratory Manual,* the genes encoding the polypeptides SEQ ID NO: 1 and SEQ ID NO:8 obtained in Example 1 were cloned into the pEB03 plasmid using restriction enzymes and T4 DNA ligase to construct recombinant expression plasmids. Specifically, the polypeptide-encoding gene fragments and the pEB03 plasmid were digested with the restriction enzymes EcoRI and NotI. The fragments and the plasmid were then ligated using T4 DNA ligase, and the ligated plasmids were transformed into *Escherichia coli* DH5α. After screening and identification, two recombinant expression plasmids pEB03-LNBP-M3 and pEB03-LNBP-M3-C236Y/D572V were obtained.

The method for transforming pEB03-LNBP-M3/pEB03-LNBP-M3-C236Y/D572V plasmids into *Bacillus subtilis* is described as follows: One inoculating loop-full of *Bacillus subtilis* glycerol stocks were streaked onto the LB agar plate (LB medium: 1.0% peptone, 0.5% yeast extract, 1.0% NaCl, and 1.5% agar powder added to the plate)) and incubated in an incubator overnight at 37°C. A single colony was selected and inoculated into 1 mL of LB medium, incubated overnight at 37°C with shaking at 200 rpm. 200 µL of the culture was transferred to 200 mL of growth medium (Growth medium: LB medium with 0.5 M sorbitol) and incubated until the OD₆₀₀ reached 0.85-0.95, was then placed on ice for 10 min, centrifuged at 5,000 rpm, 4°C for 5 min, and washed 4 times with pre-cooled electroporation medium (Electroporation medium: LB medium with 0.5 M sorbitol and 10% glycerol). Finally, the pellet was resuspended in 2 mL of pre-cooled electroporation medium. The treated pellet solution above was divided into 80 µL per tube and was added with 1 µL (approximately 50 ng) of plasmid, transferred to a pre-cooled electroporation cuvette, and placed on ice for 1-1.5 min. Next, electroporation was performed with settings of 2000 V and 200 Ω. Then the cuvette was placed on ice for 2 min, and 1 mL of recovery medium (Recovery medium: LB medium with 0.5 M sorbitol and 0.38 M mannitol) was added. The cells were incubated at 37°C with shaking at 200 rpm for 90 min. The transformed cells fluid were then plated on LB plates containing chloramphenicol (5 µg/mL) and incubated. The desired *Bacillus subtilis* recombinant strains were selected from the transformants.

Positive transformed single colonies selected from the LB plate containing 5 µg/mL of chloramphenicol were inoculated into 20 mL of seed medium (containing 0.5% yeast extract powder, 0.5% tryptone, 1% glucose, 1.8% dipotassium phosphate, and 5 µg/mL of chloramphenicol) and cultured at 37°C with shaking at 220 rpm for 8 h. Subsequently, 2.5 mL of this culture was transferred to 50 mL of fermentation medium (containing 1.0% tryptone, 0.5% yeast extract, and 1.0% NaCl) and incubated at 34°C with shaking at 250 rpm for 60 h. The supernatant was collected and analyzed by SDS-PAGE to detect the expression of the target protein. The target protein crude enzyme solution was obtained and purified according to the method as described in Example 1, and the molecular weight of the band was found to be basically consistent with the expected molecular weight of 103 kDa. The amino acid sequences of the obtained proteins were shown in SEQ ID NO: 1 or SEQ ID NO:8 and were named BS-LNBP-M3 or BS-LNBP-M3-C236Y/D572V, respectively.

The obtained BS-LNBP-M3 and BS-LNBP-M3-C236Y/D572V were used for the catalytic synthesis as described in Example 2, with the reaction system placed in a water bath at 37°C for 24 h. The yield of LNT in the reaction solution was measured using HPLC, and the results are recorded in Table 6.

**Table 6. Catalytic synthesis of LNT by BS-LNBP-M3 and BS-LNBP-M3-C236Y/D572V**

| Recombinant protein | Concentration of LNT, mg/mL |
|---|---|
| BS-LNBP-M3 | 6.47 |
| BS-LNBP-M3- C236Y/D572V | 8.76 |

### Example 6. Expression of Mature Polypeptides Sequences SEQ ID NO:1 (LNBP-M3) and SEQ ID NO:8 (LNBP-M3-C236Y/D572V) in Aspergillus oryzae (CCTCC AF 93036)

Based on the amino acid sequences of SEQ ID NO: 1 and SEQ ID NO:8, codon optimization and DNA synthesis were performed to obtain the genes of AO-LNBP-M3 and AO-LNBP-M3-C236Y/D572V. RNA was extracted from *Aspergillus oryzae.* Through reverse transcription, cDNA was produced. Using *Aspergillus oryzae* cDNA as a template, the *Aspergillus oryzae* amylase promoter PamyB and glycosidase terminator TagdA were amplified. The plasmids DCAMBIA001-AO-LNBP-M3 and pCAMBIA001-AO-LNBP-M3-C236Y/D572V were constructed via enzyme digestion and ligation. Through sequencing, the target genes were confirmed to be correctly inserted.

The preparation of competent *Agrobacterium tumefaciens* EHA105 is described as follows: 1) Activated *Agrobacterium tumefaciens* EHA105 on a solid YEB plate was inoculated into 20 mL of YEB liquid medium containing rifampicin and cultured at 28°C and 180 rpm for 24 h. 2) The *Agrobacterium tumefaciens* was inoculated at an inoculation amount of 1% into 50 mL of YEB liquid medium containing rifampicin and then cultured at 28°Cand 180 rpm until the OD₆₀₀ reached 0.8, which is ready to use. 3) The culture (50 mL) was placed on ice for 30 min, followed by centrifugation at 4°C and 5,000 rpm for 10 min, and the supernatant was discarded. 4) The cells were resuspended in 10 mL of a pre-cooled 0.1M CaCl₂ solution, placed on ice for 30 min, and then centrifuged at 4°C and 5,000 rpm for 10 min. Then, the supernatant was discarded. 5) The EHA105 cells were resuspended in 2 mL of a pre-cooled 0.1M CaCl₂ and 2 mL of glycerol. After that, the suspension was aliquoted 100 µL per tube and stored at -70°C for future use.

The freeze-thaw method used for transforming plasmid into EHA105 competent cellsis as follows: 1) *Agrobacterium tumefaciens* EHA105 competent cells were thawed on ice. Then, 10 µL of the recombinant plasmid was added to the competent cells, mixed thoroughly, and left on ice for 30 min. 2) The mixture was quickly transferred into liquid nitrogen to keep 5 min, then was immediately incubated in a 28°C water bath for 5 min. 3) The mixture was placed on ice for 5 min. 4) 400 µL of rifampicin-free YEB liquid medium was added to the mixture, followed by shaking at 28°C and 180 rpm for 2 h. 5) 200 mL of the above cultures were spread on solid YEB plates containing rifampicin and kanamycin and were incubated upside down at 28°C for 48 h to observe colony formation. 6) Single colonies were selected and cultured in 20 mL of YEB liquid medium containing rifampicin and kanamycin. The cultures were shaken at 28°C and 180 rpm for 48 h. Positive clones were screened and then the plasmid DNA was extracted and verified via sequencing.

The *Agrobacterium tumefaciens-*mediated transformation of *Aspergillus oryzae* with expression vectors is as follows: 1) *Agrobacterium tumefaciens* EHA105 bacterial culture containing different expression vectors was inoculated into LB liquid medium supplemented with rifampicin and kanamycin and incubated at 28°C with shaking at 180 rpm for 24 h. 2) The bacterial culture (2 mL) was added to a mixed medium comprising 10 mL of LB liquid medium and 10 mL of basal medium to incubate at 28°C with shaking at 180 rpm for 48 h. 3) The above culture (2 mL) was added to 20 mL of induction medium, and was shaken at 28°C and 180 rpm until the OD600 reached 0.8, at which point it was co-cultivated with *Aspergillus oryzae.* 4) A conidial suspension of *Aspergillus oryzae* was prepared. 5) 100 µL of the conidial suspension was taken and thoroughly mixed with 100 µL of the EHA105 culture. Then, the mixture was spread on a solid induction medium containing acetosyringone, which had been overlaid with a microporous membrane. The plates were incubated at 28°C for 3 days. 6) The microporous membrane was taken and spread upside down onto Czapeck-Dox agar plates containing hygromycin B and cephalosporins for antibiotic selection. These plates were incubated at 30°C for 3 days. 7) The microporous membrane was removed, and incubation continued until resistant colonies grew. Then, the resistant colonies screened were transferred to another selective medium and were cultured for screening. Resistant transformants screened were selected for fermentation verification.

The engineered *Aspergillus oryzae* strains were activated and incubated at a constant temperature of 30°C for 3 days. The spores were washed with sterile water to create a spore suspension for inoculation. Different strains were respectively inoculated into a liquid fermentation medium at an inoculation amount of 2% and cultured at 30°C and 200 rpm for 6 days. After that, the cultures were centrifuged at 12,000 rpm to collect the supernatant, which was then concentrated using a 10 kDa ultrafiltration membrane.

The crude enzyme solution containing the target proteins was purified according to the method described in Example 1. The molecular weights of the protein bands were basically consistent with the expected molecular weight of 103 kDa. The amino acid sequences of the obtained proteins matched SEQ ID NO:1 and SEQ ID NO:8, which were named AO-LNBP-M3 and AO-LNBP-M3-C236Y/D572V, respectively.

Using the obtained AO-LNBP-M3 and AO-LNBP-M3-C236Y/D572V, the catalytic synthesis of LNT was performed according to the method described in Example 2. The reaction mixtures were respectively incubated in a water bath at 37°C for 24 h. The yield of LNT in the reaction solution was measured using HPLC, and the results are recorded in Table 7.

**Table 7. Catalytic synthesis of LNT by AO-LNBP-M3 and AO-LNBP-M3- C236Y/D572V**

| Recombinant protein | Concentration of LNT, mg/mL |
|---|---|
| AO-LNBP-M3 | 6.49 |
| AO-LNBP-M3- C236Y/D572V | 8.64 |

### Example 7. Use of Mature Polypeptide (SEQ ID NO:5, LNBP-M3-N460M) for Catalytic Synthesis of LNT with Lactose as a Substrate

Lactase was purchased from Vland Biotech. Following the method described in the paper *"*Immobilization of β-Galactosidase and Its Use in Preparing Low Lactose Milk" (The Food Industry, 2018, 39(9):105-110), 20 mmol/L lactose was added to a 100 mmol/L Tris-HCl buffer (pH 6.5), and then 120 U of lactase was added per gram of lactose and thoroughly mixed. The mixture was reacted at 37°C for 5 h to obtain mixture A.

The LNBP-M3-N460M obtained in Example 1 was mixed with mixture A. According to the methods provided in Chinese Invention Patent CN201911055516.5 and the paper *"*Lacto-N-biose Synthesis via a Modular Enzymatic Cascade with ATP Regeneration" (iScience, 24(3): 102236), after the pH was adjusted to 7.0, 20 mmol/L ATP and 20 U/mL galactokinase (Galk; EC 2.7.1.6) were added. The mixture was reacted at 30°C for 60 min. After that, 10 mmol/L LNTII and 1 mmol/L MgCl₂ were added, thoroughly mixed and the mixture was reacted at 37°C for 24 h. Upon termination of the reaction, the reaction mixture was detected using IC, and the yield of LNT was 4.48 mg/mL.

### Example 8. Use of Mature Polypeptide (SEQ ID NO:5, LNBP-M3-N460M) for Catalytic Synthesis of LNT with Galactose as a Substrate

Following the methods provided in Chinese Invention Patent CN201911055516.5 and the paper *"*Lacto-N-biose Synthesis via a Modular Enzymatic Cascade with ATP Regeneration" (iScience, 24(3): 102236), 20 mmol/L galactose and 20 mmol/L ATP were added to a 100 mmol/L Tris-HCl buffer (pH 7.0) and mixed, and then 20 U/mL galactokinase (Galk; EC 2.7.1.6) was added. The reaction is at 30°C for 60 min. Subsequently, 10 mmol/L LNTII and 1 mmol/L MgCl₂ were added, along with 2.2 mg/mL of the mature polypeptide LNBP-M3-N460M obtained in Example 1 were added and mixed. The mixture was then reacted at 45°C for 0.5 h. Upon termination of the reaction, the reaction mixture was detected using IC, and the yield of LNT was 5.07 mg/mL.

It should be noted that the aforementioned examples are only intended to illustrate the technical solutions hereof and are not meant to limit the present invention. Although the present invention has been described in detail regarding the given examples, those skilled in the art may make modifications or equivalent replacements to the technical solutions as needed without departing from the spirit or scope of the technical solutions.

## Claims

1. A mature polypeptide sequence, **characterized by** modifications comprising one or more of the following modifications (1) to (4):
(1) substituting cysteine at position 236 in the sequence shown in SEQ ID NO: 1 with tyrosine or valine;
(2) substituting asparagine at position 460 in the sequence shown in SEQ ID NO:1 with isoleucine, valine, or methionine;
(3) substituting aspartic acid at position 572 in the sequence shown in SEQ ID NO: 1 with valine or methionine;
(4) substituting serine at position 608 in the sequence shown in SEQ ID NO:1 with valine or isoleucine;
preferably, the modifications are:
the cysteine at position 236 in the sequence shown in SEQ ID NO:1 is substituted with tyrosine, thereby obtaining the amino acid sequence shown in SEQ ID NO:2;
the asparagine at position 460 in the polypeptide sequence shown in SEQ ID NO:1 is substituted with isoleucine, valine, and methionine, thereby obtaining the amino acid sequences shown in SEQ ID NO:3, SEQ ID NO:4, and SEQ ID NO:5, respectively;
the aspartic acid at position 572 in the polypeptide sequence shown in SEQ ID NO: 1 is substituted with valine, thereby obtaining the amino acid sequence shown in SEQ ID NO:6;
the serine at position 608 in the polypeptide sequence shown in SEQ ID NO: 1 is substituted with valine, thereby obtaining the amino acid sequence shown in SEQ ID NO:7;
the cysteine at position 236 in the polypeptide sequence shown in SEQ ID NO:1 is substituted with tyrosine, and simultaneously, aspartic acid at position 572 is substituted with valine, thereby obtaining the amino acid sequence shown in SEQ ID NO:8;
cysteine at position 236 in the polypeptide sequence shown in SEQ ID NO:1 is substituted with tyrosine, and simultaneously, serine at position 608 is substituted with valine, thereby obtaining the amino acid sequence shown in SEQ ID NO:9;
cysteine at position 236 in the polypeptide sequence shown in SEQ ID NO:1 is substituted with tyrosine, simultaneously, asparagine at position 460 is substituted with methionine, and serine at position 608 is substituted with valine, thereby obtaining the amino acid sequence shown in SEQ ID NO:10.

2. A polynucleotide encoding the polypeptide according to claim 1.

3. A nucleic acid construct comprising the polynucleotide according to claim 2, and one or more regulatory sequence(s) that can be operatively linked to the polynucleotide, and these regulatory sequence(s) can direct the production of the polypeptide in an appropriate expression host.

4. An expression vector comprising the polynucleotide according to claim 2 or the nucleic acid construct according to claim 3.

5. A transformed host cell transformed with the nucleic acid construct according to claim 3 or the expression vector according to claim 4.

6. A composition **characterized by** containing the mature polypeptide sequence according to claim 1.

7. An engineered bacterium **characterized by** containing the mature polypeptide sequence according to claim 1, wherein the host cell of the engineered bacterium is selected from yeast cells, filamentous fungal cells, and bacterial cells;
preferably, the bacterial cells are selected from *Escherichia sp.* or *Bacillus sp*.;
preferably, the cells of *Escherichia sp.* are selected from *Escherichia coli*.;
preferably, the cells of *Bacillus sp.* are selected from *Bacillus mycoides, Bacillus cereus, Bacillus polymyxa, Bacillus licheniformis, Bacillus brevis, Bacillus circulans,* and *Bacillus subtilis*;
more preferably, the host cells are selected from *Bacillus circulans* or *Bacillus subtilis*;
preferably, the filamentous fungal cells are selected from *Aspergillus sp.* and *Trichoderma sp*.;
the cells of *Aspergillus sp.* are selected from (*Aspergillus oryzae, Aspergillus fumigatus, Aspergillus niger,* and *Aspergillus flavus*;
more preferably, the host cells are cells of *Aspergillus oryzae*;
preferably, the yeast cells include *Candida sp., Hansenula sp., Kluyveromyces sp., Pichia sp., Saccharomyces sp., Schizosaccharomyces sp.,* and *Yarrowia sp*.;
more preferably, the said yeast cells are *Saccharomyces cerevisiae, Kluyveromyces lactis,* or *Kluyveromyces marxinus.*

8. The engineered bacterium according to claim 7, **characterized in that** the engineered bacterium is *Kluyveromyces lactis,* or *Bacillus subtilis,* or *Aspergillus oryzae.*

9. The application of the polypeptide according to claim 1 or a composition containing the polypeptide in producing lacto-N-tetraose (LNT) process;
preferably, the application includes the catalytic synthesis of lacto-N-tetraose (LNT) using the polypeptide according to claim 1 or a composition containing the polypeptide.

10. A method for producing lacto-N-tetraose (LNT) that uses the polypeptide according to claim 1, composition according to claim 6, or engineering bacterium according to claims 7;
preferably, the method includes the use of the polypeptide according to claim 1, composition according to claim 6, or engineering bacterium according to claim 7 and substrate in the reaction solvent to catalyze the synthesis or fermentation synthesis of lacto-N-tetraose (LNT);
preferably, the substrate for the reaction is selected from Galactose-1-phosphate, lacto-N-triose II (LNTII), lactose, galactose, acetylglucosamine, and acetylglucosamine group-containing carbohydrates;
preferably, the substrate for the reaction is lacto-N-triose II (LNTII);
preferably, the reaction solvent is an aqueous solvent or a solution containing solids;
preferably, the polypeptide is in the form of a culture medium containing the polypeptide or a purified polypeptide.
